# EUROPÄISCHE PATENTSCHRIFT

(11) **EP 1 764 716 B1**
(45) Veröffentlichungstag und Bekanntmachung des Hinweises auf die Patenterteilung: **26.03.2008**
(21) Anmeldenummer: 05020406.4
(22) Anmeldetag: 19.09.2005
(51) Int. Cl.: G06F 19/00, A61M 5/14, A61M 5/172

(54) **Verfahren und Vorrichtung zum Planen einer Direktinfusion in Lebergewebe**
Device and method for planning a direct infusion into liver tissue
Appareil et procédé pour planifier une infusion directe dans un tissu hépatique

(43) Veröffentlichungstag der Anmeldung: 21.03.2007
(73) Patentinhaber: BrainLAB AG, 85551 Kirchheim/Heimstetten (DE)
(72) Erfinder: Hartlep, Andreas, Dr., 83629 Naring (DE); Pedain, Christoph, Dr., 81667 München (DE)
(74) Vertreter: Gassenhuber, Andreas

(56) Entgegenhaltungen:
- EP-A- 1 398 641
- US-A1- 2002 188 275
- US-A1- 2003 114 751
- US-B1- 6 722 370

## Beschreibung

Die vorliegende Erfindung betrifft ein Verfahren und eine Vorrichtung zum Planen einer Infusion, insbesondere einer Direktinfusion, in Lebergewebe.

Um die Leber bei einer Erkrankung, wie zum Beispiel Leberkrebs, zu behandeln, können Medikamente direkt in das Zielgewebe abgegeben werden. Da die Vaskularisation der Leber groß ist und diese somit von vielen Gefäßen durchzogen wird, ist der Abfluss von in die Leber zugeführten Substanzen in den restlichen Körper sehr hoch. Es existieren Techniken, um die Perfusion des Lebergewebes zu beeinflussen. Wenn die Perfusion reduziert werden kann, kann die Verweildauer der verabreichten Substanz in der Leber verlängert werden und demzufolge kann der therapeutische Effekt bei gleichzeitiger Verringerung der Nebenwirkungen verbessert werden.

Zur Behandlung von Leberkrebs ist eine Transarterielle Chemoembolisation (TACE; transarterial chemoembolization) genannte Technik bekannt, wobei bei diesem Verfahren die Blutzufuhr zu einem Tumor unterbrochen wird und die Chemotherapie direkt an den Tumor verabreicht wird. Dabei werden zum Beispiel die chemotherapeutischen Mittel in die hepatitische (Leber-) Arterie injiziert, welche zu dem Lebertumor führt, Bei Chemoembolisation wird zusätzliches Material injiziert, um die kleinen Zweige der Hepatitischen Arterie zu blockieren. Es ist jedoch sehr wahrscheinlich, dass ein beträchtlicher Teil der chemotherapeutischen Mittel in den Rest des Körpers gelangt. Deshalb kann eine selektive intraarterielle Chemotherapie die gewöhnlichen systemischen, sich auf ganzen Körper auswirkenden Nebeneffekte verursachen. Zusätzlich kann diese Behandlung zu einigen sich nur regional auf bestimmte Körperteile auswirkende Nebeneffekten fuhren, wie zum Beispiel eine Entzündung der Gallenblase (Cohlecystitis), Darm- und Magengeschwüre und eine Entzündung des Pankreas (Pankreatitis). Bei Patienten mit einem hepatozellularen Karzinom (HCC) mit einer fortgeschrittenen Zirrhose kann es nach einer solchen Behandlung auch zu einem Leberversagen kommen.

Andere Verfahren zur Behandlung der Leber umfassen das Verbinden von radioaktivem Material mit Antikörpern, welche auf bestimmte Zielgebiete in Leberkrebszellen angesetzt werden (Immunotherapie).

Aus der EP 1316 324 A1 der Anmelderin ist ein Verfahren und eine Vorrichtung zur Verabreichung einer Substanz bekannt.

Es ist eine Aufgabe der vorliegenden Erfindung ein Verfahren und eine Vorrichtung vorzuschlagen, welche eine verbesserte Behandlung von Lebergewebe ermöglichen.

Diese Aufgabe wird durch die Gegenstände der unabhängigen Patentansprüche gelöst. Vorteilhafte Ausführungsformen ergeben sich aus den abhängigen Ansprüchen.

Bei dem erfindungsgemäßen nicht chirurgischen und nicht therapeutischen Verfahren zum Planen einer Infusion, insbesondere einer Direktinfusion, in die Leber oder in Lebergewebe werden individuell für einen zu behandelnden Patienten anatomische und/oder physiologische Patientendaten erfasst. Beispielsweise können Patientendaten oder daraus gewonnene Patientenparameter durch bekannte Kemspinresonanzverfahren (MRI), Cotnputertomographie (CT)-Verfahren, PET, SPECT, Röntgen-, Ultraschall-Verfahren gewonnen werden, welche es ermöglichen zum Beispiel die räumliche Stuktur eines Körpers, insbesondere der Gewebestruktur eines Körpers, wie zum Beispiel der Leber und des umgebenden Gewebes zu erfassen und darzustellen und/oder funktionelle Daten, wie zum Beispiel patientenspezifische Diffusions- und Perfussionseigenschaften oder Daten bezüglich des Blutdrucks zu gewinnen. Ergänzend können auch zum Beispiel vorher in einer Datenbank abgespeicherte Daten verwendet werden, um zum Beispiel Daten, welche mittels eines bildgebenden Verfahrens gewonnen wurden, auszuwerten und zum Beispiel bestimmten Gewebetypen, Strukturen oder Eigenschaften, wie zum Beispiel einen Durchblutungsgrad, zuzuordnen.

Unter Verwendung der ermittelten Patientendaten wird erfindungsgemäß die Durchführung der Leberinfusion geplant, d. h. es werden unter Berücksichtigung verschiedener auswählbarer Vorgaben, wie zum Beispiel den Patientendaten oder auch Parametern von zur Verfügung stehenden zu verabreichenden Substanzen, wie zum Beispiel chemotherapeutischen Mitteln (Medikament, Arznei, Toxin, Microspheres, wie zum Beispiel Kapseln aus zum Beispiel Eiweißes, die einen Wirkstoff enthalten können und zum Beispiel eine Größe im sub-mm bis nano-Bereich haben, Liposomen, radioaktive Substanzen, Gase, Hormone,...), Parametern der verwendbaren Katheter und gegebenenfalls einer verwendbaren Pumpe, beispielsweise eine geeignete Auswahl einer vor dem Infusionsmittel verabreichten physiologisch wirksamen Substanz und/oder des zu verwendenden Infusionsmittels und/oder Katheters getroffen, die Positionierung des Katheters hinsichtlich Anbringungsort und Eindringtiefe simuliert oder geplant, die Substanz und/oder das Infusionsmittel falls erforderlich modifiziert, zum Beispiel in der Konzentration verändert, wie zum Beispiel verdünnt, und zum Beispiel der zeitliche Verlauf des Druckes, mit welchem die Substanz und/oder das Infusionsmittel durch einen oder mehrere Katheter zugeführt werden soll, vorgegeben. Ziel einer solchen Auswahl und Einstellung ist es, eine definierte Menge der mit dem Infusionsmittel zu verabreichenden Substanz in ein Zielgewebevolumen der Leber einzubringen, um dort eine bestimmte Konzentration zu erhalten, wobei so wenig der zu verabreichenden Substanz wie möglich in Nicht-Zielgewebe eingebracht werden soll.

Es können zum Beispiel der Zeitverlauf der Substanz- und/oder Infusionsmittelverteilung zum Beispiel in der Leber und/oder die Aufnahme des Infusionsmittels durch oder in Gewebe und/oder die Wechselwirkung mit anderen verabreichten Substanzen berechnet werden und zum Beispiel zu verschiedenen Zeitpunkten dargestellt werden.

Durch die Verwendung von zwei oder mehr gemeinsam oder separat verabreichten Substanzen oder Infusionsmitteln können die physiologischen Eigenschaften, wie zum Beispiel die Durchblutung eines Gewebebereichs beeinflusst werden, um zum Beispiel die Verweildauer eines anschließend verabreichten therapeutischen Mittels in dem zu behandelnden Gewebe zu verlängern.

Erfindungsgemäß kann die Verteilung eines Infusionsmittels und einer Substanz, welche Transportprozesse in dem Zielbereich oder Zielgewebe beeinflusst, berechnet werden, wie zum Beispiel die Durchblutung oder der Blutfluss und/oder die Perfusion in der Leber oder die Auswirkung einer vor dem Infusionsmittel verabreichten die physiologischen Eigenschaften beeinflussenden Substanz hierauf. Die Effekte, also zum Beispiel mögliche Wirkungen eines Infusionsmittels und einer vor dem Infusionsmittel verabreichten physiologisch wirksamen Substanz und der Bereich oder die Bereiche, welche von diesen Effekten zum Beispiel nach der Verabreichung der Substanz zu verschiedenen Zeitpunkten betroffen sind, können berechnet werden, wie zum Beispiel eine Verringerung des Blutflusses in einem bestimmten Bereich der Leber zum Beispiel durch die infundierte Substanz, um die Wirkungszeit und/oder die Konzentration des Infusionsmittels in einem bestimmten Bereich zu erhöhen. Basierend auf der Berechnung der Verteilung eines therapeutischen Mittels, und der Verteilung oder Auswirkung eines Effektes des therapeutischen Mittels kann die Wirkung des Mittels auf ein Gewebe simuliert werden und optional die Infusionsplanung verändert werden.

Somit ist es erfindungsgemäß möglich die Verteilung von Substanzen, welche direkt in Lebergewebe verabreicht werden, oder die Auswirkungen einer solchen Substanzenverteilung und damit einen Behandlungserfolg durch die Verabreichung solcher Substanzen zu berechnen und zu simulieren. Hierdurch wird es einem Arzt ermöglicht das Ergebnis einer Behandlung basierend auf der direkten Verabreichung von Substanzen, wie zum Beispiel Arzneimitteln und/oder einer Energie, wie zum Beispiel Wärme und/oder basierend auf einer Simulation der Substanzverteilung zu bestimmen, um zu einen optimierten Behandlungsplan für einen Patienten basierend auf dessen individueller Anatomie und physiologischer Eigenschaften zu erstellen, was zu einer Optimierung der Behandlungseffekte und somit einem größeren Behandlungserfolg bei gleichzeitiger Verringerung von Nebenwirkungen führt. Folglich ist es nicht mehr wie im Stand der Technik erforderlich zum Beispiel die Aufnahme der Substanz durch Gewebe basierend auf der individuellen Erfahrung eines Arztes abzuschätzen, sondern eine Behandlung kann unter Verwendung von bekannten Wirkungsmechanismen individuell für einen Patienten optimiert werden.

Zur Positionierung der Infusionsvorrichtung(en), beispielsweise eines oder mehrerer Katheter, können die erfassten Patientendaten verwendet werden, wobei aus diesen Patientendaten ermittelt wird, wo genau sich im Körper des Patienten ein zu behandelndes Gewebevolumen, wie zum Beispiel ein Lebertumor befindet. Unter Verwendung dieser Information kann ein geeigneter Katheter zum Beispiel aus einer vorhandenen Datenbank durch einen Benutzer oder vollautomatisch ausgewählt werden und gegebenenfalls durch Nachbearbeitung, zum Beispiel zurechtschneiden der Katheterlänge, entsprechend anwendungs- oder patientenspezifisch zum Beispiel bezüglich der gewünschten Eindringtiefe in Gewebe modifiziert werden. Weiterhin kann ermittelt werden, wo eine geeignete Stelle zum Anbringen des Katheters ist, um möglichst wenig gesundes Gewebe durch die Infusion zu belasten.

Vorteilhaft können bekannte Verfahren zur Positionierung zum Beispiel unter Verwendung von reflektierenden Markern, welche am Katheter angebracht werden und von IR-Kameras detektiert werden oder von magnetischen Spulen, die ein definiertes externes magnetisches Feld detektieren, verwendet werden, um den Katheter an einer gewünschten Position des Patienten anzubringen. Dabei können am Patienten selbst ebenfalls Marker angebracht sein, welche als Referenz dienen und durch welche ein Patientenkoordinatensystem festgelegt wird, in welchem der Katheter an einer bestimmten ermittelten Stelle platziert wird.

Bevorzugt werden unter Verwendung der erfassten Patientendaten zur Planung der Infusion patientenspezifische Parameter ermittelt, wie zum Beispiel die Gewebe- oder Körperstruktur im Bereich des durch eine Infusion zu behandelnden Gewebes. Insbesondere ist es vorteilhaft, die Gewebedichte, die Verteilung bestimmter Gewebestrukturen, oder die Durchblutung eines bestimmten Gewebebereiches als Patientenparameter zu ermitteln. Patientenparameter können sowohl unmittelbar aus den erfassten Patientendaten, als auch aus Datenbanken oder einer Kombination aus in Datenbanken gespeicherten Werten zusammen mit den erfassten Patientendaten gewonnen werden. So können beispielsweise in Datenbanken Werte bezüglich der gewöhnlichen Durchblutung bestimmter Gewebebereiche, das Diffusions- und Perfusionsverhalten ausgewählter Substanzen in dem betrachteten Gewebe, Werte bezüglich des Gewebeverhaltens nach Zufuhr einer bekannten Substanz, zum Beispiel durch Anschwellen des Gewebes oder Stoffwechselreaktionen gespeichert werden, welche als Patientenparameter zur Planung einer Infusion verwendet werden können.

Es ist weiterhin vorteilhaft, Infusionsmittelparameter zu ermitteln, welche charakteristisch für die zu verabreichende Substanz oder einen Wirkstoff sind und beispielsweise die physikalischen, chemischen und/oder biologischen Eigenschaften definieren. So kann beispielsweise aus einer Datenbank eine Information bezüglich der Molekül- oder Teilchengröße der zu verabreichenden Substanz, der Diffusionsgeschwindigkeit dieser Substanz in einer bestimmten Gewebeart, den Metabolismus bzw. die Wechselwirkung der Substanz mit Gewebe aufgrund von Stoffwechselprozessen, ein für die Substanz für den zu behandelnden Gewebetyp bekannten Diffusionskoeffizienten oder vorteilhaften Injektionsdruck oder Druck-Gradienten, eine vorteilhafte Konzentration, Menge oder Zufuhrrate, deren Größenordnung gewöhnlich im Bereich ml/h liegt, gewonnen werden. Die beispielhaft aufgezählten Infusionsmittelparameter können einzeln oder in Kombination zusammen mit anderen Parametern zur Planung der Infusion verwendet werden.

Vorteilhaft werden Katheterparameter, d. h. für einen Katheter spezifische Größen zur Planung der Infusion verwendet, wobei verschiedene Kathetertypen zum Beispiel in einer Datenbank zur Auswahl zur Verfügung gestellt werden können. Für die Infusion relevante Katheterparameter können zum Beispiel der Innendurchmesser des Katheters, Oberflächenbeschaffenheit, das Material, insbesondere die Steifigkeit des Katheters, die Anzahl und Anordnung der Austrittsöffnungen an dem Katheter oder eine bekannte Eignung eines bestimmten Kathetertyps für eine bestimmte zu verabreichende Substanz oder einen bestimmten zu behandelnden Gewebetyp oder Gewebeerkrankung sein. Allgemein können erfindungsgemäß auch mehrere Katheter verwendet werden.

Als Infusionsmittel kann erfindungsgemäß beispielsweise ein Medikament, ein Toxin, Microspheres, Liposomen, radioaktive Substanzen, Gase, Hormone, eine Lösung mit Zellen, Viren, Genen, Enzymen, Proteinen, Hormonen, Antikörpern oder einer Kombination daraus verwendet werden.

Unter Verwendung der oben beispielhaft aufgeführten Patientenparameter, Infusionsmittelparameter und/oder Katheterparameter einzeln oder in Kombination, zusammen mit den erfassten Patientendaten kann eine durchzuführende Infusion erfindungsgemäß so geplant werden, dass ein möglichst großer Anteil einer Substanz in einen Zielgewebebereich der Leber durch Infusion eingebracht wird, wobei möglichst wenig der Substanz in Nicht-Zielgewebe abgegeben wird. Eine durch Infusion in Gewebe einzubringende Substanz kann erfindungsgemäß somit mit einem besonders geeigneten und richtig positionierten Kathetertyp und dem richtigen Injektionsdruck in einer geeigneten Konzentration mit einer gewünschten Rate unter Berücksichtigung von Stoffwechsel- und Diffusionsprozessen in einen zu behandelnden Gewebebereich eines Patienten eingebracht werden, um in diesem Gewebebereich eine gewünschte Konzentration der einzubringenden Substanz zu erhalten. Umliegendes Gewebe wird dabei so wenig wie möglich belastet.

Vorteilhaft kann erfindungsgemäß zur Vorwärts-Planung der Infusion eine Simulation der durchzuhihrenden Infusion, zum Beispiel durch Berechnung der Infusionsmittelverteilung in Gewebe unter Verwendung der erfassten Patientendaten und der oben genannten verschiedenen Parameter durchgeführt werden. Durch eine solche Simulation kann die Infusionsmittelverteilung sowohl statisch als auch dynamisch als Funktion der Zeit ermittelt und vorteilhaft graphisch dargestellt werden. Somit kann bereits vor Durchführung einer Infusion festgestellt werden, ob eine gewünschte Konzentrationsverteilung der einzubringenden Substanz in dem Zielgewebe erhalten werden kann, oder ob gegebenenfalls Infusionsmittelparameter, Katheterparameter oder Positionsparameter verändert werden müssen, um einen besseren Erfolg der Infusion sicherzustellen.

Weiterhin kann erfindungsgemäß auch eine Rückwärts- oder inverse Planung erfolgen, wobei zum Beispiel von einer Bedienperson bestimmte Behandlungsdaten vorgegeben werden, wie zum Beispiel das zu behandelnde Zielvolumen, vorteilhaft zusammen mit Risikostrukturen wie zum Beispiel Nervenbahnen, welche nicht durch die Infusion beeinträchtigt werden sollten und die Angabe des zu behandelnden Gewebetyps. Dabei kann entweder vollautomatisch oder in Wechselwirkung mit dem Benutzer, zum Beispiel durch Anzeige eines Auswahlmenüs, der Ablauf der Infusion festgelegt werden, d. h. es können ein oder mehrere Kathetertypen zusammen mit geeigneten Infusionstnitteln ausgewählt, die Katheteranordnung(en) hinsichtlich Position und/oder Eindringtiefe festgelegt und die Infusionsmittelparameter eingestellt werden, um eine möglichst optimale Infusionsbehandlung für das vorgegebene Zielvolumen zu ermöglichen.

Die oben beschriebenen Planungsverfabren, insbesondere die Auswahl der einzelnen Parameter, können vollautomatisch zum Beispiel unter Verwendung von in Datenbanken gespeicherten Werten, halbautomatisch zum Beispiel durch noch von einem Benutzer zu treffende Auswahlen aus einem angezeigten Menü oder manuell zum Beispiel durch von einem Benutzer einzugebende Parameterwerte erfolgen. Vorteilhaft werden hierbei geeignete Rechner zusammen mit Eingabe- und Ausgabe-, zum Beispiel Anzeigeelementen verwendet, welche auszuwählende Elemente, Gewebestrukturen, berechnete Konzentrationsverteilungen des Infusionsmittels im Gewebe und weitere Informationen anzeigen.

Gemäß einem weiteren Aspekt bezieht sich die vorliegende Erfindung auf ein Computerprogramm, welches, wenn es in einen Computer geladen ist oder auf einem Computer läuft, das oben beschriebene Verfahren ausführt. Ebenso bezieht sich die vorliegende Erfindung auf ein Speichermedium für ein solches Programm oder ein Computerprogrammprodukt mit dem oben erwähnten Programm.

Eine erfindungsgemäße Vorrichtung zur Planung einer Leberinfusion weist ein Planungssystem auf, welches aus einem Computersystem, bevorzugt mit Eingabe- und Ausgabevorrichtungen und zugehöriger Software besteht. Dabei ist vorteilhaft ein Monitor zur Anzeige von durch den Computer aus Datenbanken vorgegebenen Elementen oder aus Berechnung ermittelten Werten oder räumlichen Verteilungen vorgesehen.

Vorteilhaft ist auch ein Navigationssystem vorgesehen, welches zum Beispiel aus an zu positionierenden Elementen angebrachten reflektierenden Markern, Leuchtdioden oder Spulen und TR-Kameras oder Magnetfeldgeneratoren besteht, mit welchen unter Verwendung einer geeigneten bekannten Software und Hardware eine genaue Positionierung zum Beispiel eines Katheters an einem Körper erfolgen kann.

Allgemein umfasst die erfindungsgemäße Vorrichtung Elemente, Vorrichtungen und Systeme, mit denen die oben beschriebenen Verfahrensschritte durchgeführt werden können.

Es wird auch ein Leberinfusionsverfahren offenbart wobei die Infusion bevorzugt wie oben beschrieben vorbereitet wird und anschließend das Infusionsmittel in den Körper bzw. das Lebergewebe eingebracht wird.

Vorteilhaft wird kontinuierlich während der Infusion oder in bestimmten Zeitabständen eine Verifikation oder Überprüfung durchgeführt, wobei mit einem geeigneten Datenerfassungs- oder Abbildungssystem die Verteilung des Infusionsmittels in dem Gewebe während oder nach dem Infusiansvorgang ermittelt wird. Hierzu können zum Beispiel Kernspinresonanzoder Ultraschall-Verfahren verwendet werden, wobei es vorteilhaft sein kann, dem Infusionsmittel ein Kontrastmittel hinzuzufügen, um die Verteilung des Infusionsmittels im Körpergewebe eindeutig feststellen oder messen zu können.

Bevorzugt werden Abweichungen der bei dem Verifikations-Vorgang ermittelten tatsächlichen Infusionsmittelverteilung im Gewebe von vor oder während der Infusion ermittelten Planungsdaten ermittelt und bevorzugt angezeigt. Vorteilhaft erfolgt eine Korrektur der Infusionsparameter, d. h. eine Veränderung der chemischen und/oder physikalischen Zusammensetzung bzw. Eigenschaften des Infusionsmittel und/oder eine Veränderung der Zufuhr, zum Beispiel eine Veränderung des Injektionsdruckes oder der Abgabemenge, um die bei der Verifikation ermittelte Abweichung von der geplanten Verteilung korrigieren zu können. Falls erforderlich, kann auch ein Katheter neu positioniert oder ausgewechselt werden.

Die Verifikation und die Ermittlung der Abweichung und Korrektur erfolgt vorteilhaft in Echtzeit, so dass über eine Rückkopplung die Infusion geregelt durchgeführt werden kann, um den gewünschten Infusionserfolg zu erhalten, d. h. das Infusionsmittel wie gewünscht dem vorgegebenen Zielbereich zuzuführen.

Es wird auch ein Computerprogramm offenbart, welches, wenn es in einen Computer geladen ist oder auf einem Computer läuft, das oben beschriebene Verfahren ausführt. Ebenso bezieht sich die vorliegende Erfindung auf ein Speichermedium für ein solches Programm oder ein Computerprogrammprodukt mit dem oben erwähnten Programm.

Es wird auch eine Vorrichtung offenbart, zur Durchführung eines Infusionsverfahrens wie oben beschrieben mit einer Verifikationsvorrichtung zum Ermitteln der räumlichen Verteilung eines Infusionsmittels in einem Körper, insbesondere in einem Gewebebereich. Die Verifikationsvorrichtung kann beispielsweise ein Kernspinresonanz-, Röntgen- oder ein Ultraschall-System sein, mit welchem das Infusionsmittel oder dessen Verteilung und Konzentration im Gewebe detektiert werden kann.

Vorteilhaft ist ein Computersystem bevorzugt mit einer Anzeigevorrichtung vorgesehen, um die ermittelte räumliche Verteilung des lnfusionsmittels im Gewebe auszuwerten, eine Abweichung von einem vorher festgelegten Infusionsplan festzustellen und gegebenenfalls automatisch eine Änderung von Infusionsparametern durchzurühren oder einer Bedienperson eine solche A̅nderung vorzuschlagen, um die Infusion so zu modifizieren, dass diese wie geplant durchgeführt werden kann. Dazu können zum Beispiel Systeme vorgesehen sein, mit welchen die Konzentration des Infusionsmittels verändert werden kann und/oder der Injektionsdruck oder die Injektionsmenge zum Beispiel mittels einer Pumpe geändert werden kann, um eine Infusionsmittolverteilung im Gewebe wie vorher geplant zu erhalten. Vorteilhaft wird die Art und Größe der Veränderung der Infusionsparameter bei einer bei der Verifikation festgestellten Abweichung von einem vorgegebenen Infusionsplan unter Verwendung von bekannten Wirk- und Funktionsmechanismen ermittelt. Zum Beispiel wird die Zufuhrrate oder der Injektionsdruck verringert, wenn festgestellt wird, dass sich das Infusionsmittel schneller als vorausbestimmt ausbreitet oder nicht so schnell wie erwartet durch Stoffwechselprozesse abgebaut wird.

Die Erfindung wird nachfolgend anhand bevorzugter Ausführungsbeispiele beschrieben. Dabei zeigen:
- Figur 1: schematisch ein Schaubild eines Verfahrens zur Planung und Durchführung einer Leberinfusion;
- Figur 2: ein vereinfachtes Ablaufdiagramm einer durchgeführten Infusion; und
- Figur 3: eine Vorrichtung, welche bei der Planung und Durchführung einer Infusion verwendet werden kann.

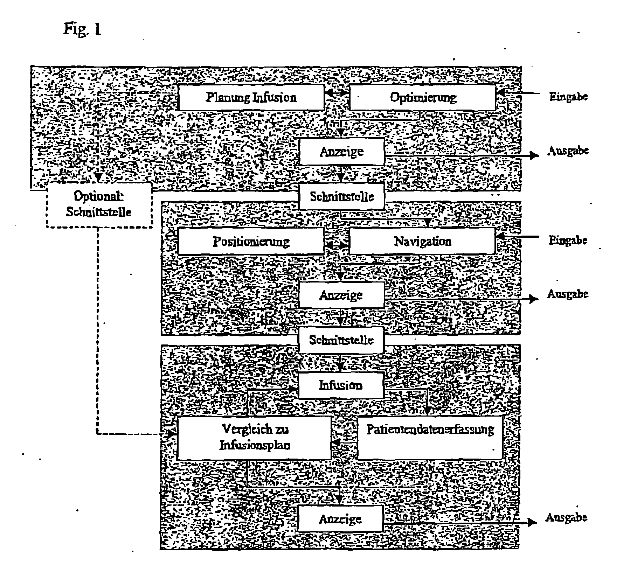

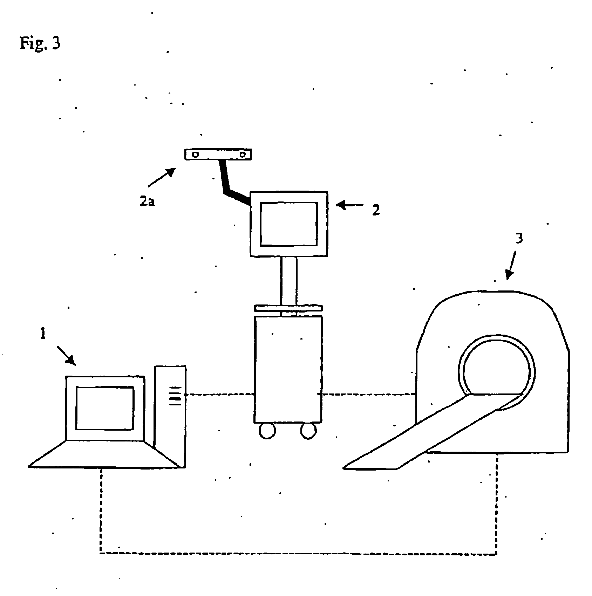

Figur 1 zeigt schematisch ein Ablaufdiagramm für die erfindungsgemäße Vorbereitung und Durchführung einer Leberinfusion. Wie in Figur 1 gezeigt, werden Patientendaten zum Beispiel von einem Kemspintomographen eingegeben, welche verwendet werden, um einen bestimmten Gewebe-Zielbereich der Leber zum Beispiel in oder in der Nähe eines Lebertumors für die Infusion zu ermitteln und die zuzuführende Infusionsdosis zu planen. Diese Daten können zum Beispiel durch das Kemspinresonanz-System 3, wie in Figur 3 schematisch gezeigt, nach der Untersuchung eines zu behandelnden Patienten gewonnen werden. Unter Verwendung von zum Beispiel in Datenbanken abgespeicherten Parametern für die Eigenschaften der Gewebestrukturen, Infusionsmittel und für verschiedene Kathetertypen kann nach der Ermittlung der genauen Lage des zu behandelnden Gewebevolumens ein oder mehrere für die Infusion geeignete Infusionsmittel und/oder Katheter ausgewählt werden. Die durch zum Beispiel das Kernspinresonanz-Verfahren gewonnenen Patientenparameter können zusammen mit den Katheteiparametem und den zum Beispiel auch in Datenbanken gespeicherten Infusionsmittelparametern verwendet werden, um die Infusion zu planen. Dabei können die entsprechenden Parameter unter der Randbedingung optimiert werden, dass ein möglichst großer Anteil des Infusionsmittels mit einer gewünschten Konzentration in das Lebergewebe eingebracht wird, wobei möglichst wenig Infusionsmittel in ein außerhalb des Lebergewebes liegendes Gewebe gelangen soll. Allgemein sollten möglichst wenige Katheter oder Nadeln platziert werden, welche durch möglichst wenige Zugänge zugeführt werden. Diese optimierte Planung der Infusionsdosis wird über eine Anzeige ausgegeben, so dass zum Beispiel eine zweidimensionale oder dreidimensionale Darstellung durch Abbildung verschiedener Schnittebenen ausgegeben werden kann, um das Ergebnis der Infusionsplanung anzuzeigen.

Der so erstellte Infusionsplan wird über eine Schnittstelle an ein Navigationssystem übermittelt, wie beispielsweise das in Figur 3 schematisch gezeigte VectorVision-System, um basierend auf den Planungsdaten den oder die ausgewählten Katheter an den vorgegebenen Stellen am Körper zu positionieren. Die Positionierung kann automatisch zum Beispiel unter Verwendung eines Roboters, oder durch das Navigationssystem geführt, von Hand erfolgen, wobei auf einer Anzeigevorrichtung dargestellt werden kann, ob ein Katheter richtig positioniert ist oder noch in eine bestimmte Richtung bewegt werden muss.

Nach erfolgter Positionierung des oder der Katheter(s) wird die eigentliche Infusion mit den durch die Planung vorgegebenen Infusionsmittelparametern durchgeführt. Dabei wird wiederum eine Patientendatenerfassung durchgeführt, um die tatsächliche Verteilung des Infusionsmittels im Lebergewebe zu ermitteln. Unter Verwendung der durch die Planung vorgegebenen Parameter und der darauf basierenden Simulationsergebnisse für die Infusion wird ein Vergleich zwischen tatsächlicher Infusionsmittelverteilung und vorausbestimmter gewünschter Infusionsmittelverteilung durchgeführt und gegebenenfalls werden die Parameter, wie zum Beispiel die Konzentration des Injektionsmittels, die Abgabemenge oder der Injektionsdruck zur Durchführung der Infusion bevorzugt unter Berücksichtigung von bekannten Wirkmechanismen abgeändert, um das gewünschte, geplante Infusionsergebnis zu erhalten. Wiederum kann die gemessene tatsächliche Verteilung der InfusionsmittelKonzentration bevorzugt zusammen mit eventuellen Abweichungen und Korrekturverfahren über eine Anzeige ausgegeben werden, um es zum Beispiel einer Bedienperson zu ermöglich manuell in das Injektionsverfahren einzugreifen.

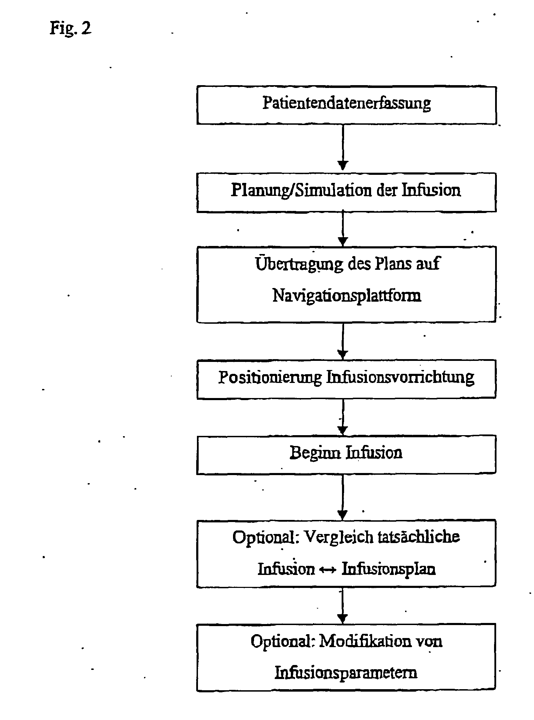

Figur 2 zeigt schematisch einen vereinfachten Ablauf einer erfindungsgemäßen Planung und Durchführung einer Leberinjektion. Zunächst werden Patientendaten durch ein bildgebendes diagnostisches Verfahren, wie beispielsweise ein Kemspinresonanz-Verfahren erfasst, um die aktuellen Patientenparameter wie zum Beispiel Gewebedichte, Durchblutung und Lage eines zu behandelnden Lebergewebes zu erhalten. Unter Verwendung der so ermittelten Patientenparameter sowie mit aus einer Datenbank erhaltenen und/oder für eine bestimmte Infusion vorgegebenen Katheter- und Infusionsmittelparametern wird die Infusion geplant und/oder simuliert. Basierend auf den so ermittelten Parameterdaten wird der Infusionsplan an eine Navigationsplattform weitergegeben, mit welcher der oder die Katheter wie im Infusionsplan vorgesehen am Patienten positioniert wurden sollen. Die Infusion beginnt nach der Positionierung der Iufusionsvorrichtung und wird mit den geplanten und gegebenenfalls simulierten Parametern durchgeführt, wobei optional, wie in Figur 1 und 2 gezeigt, ein Vergleich der tatsächlich durchgeführten Infusion mit dem Infusionsplan durchgeführt wird und bei Abweichungen eine Modifikation der entsprechenden Parameter bevorzugt unter Ausnutzung bekannter Wirkmechanismen vorgenommen wird.

Figur 3 zeigt schematisch eine Vorrichtung, welche zur Planung und Durchführung einer Infusion verwendet werden kann. Patientendaten werden in einem Kernspintomographen 3 erhalten und an ein Planungssystem 1 sowie ein Navigationssystem 2 weitergegeben. Mit dem Navigationssystem 2 werden unter Verwendung von zum Beispiel bekannten an einem oder mehreren Kathetern angebrachten Reflektoren oder Markern der oder die Katheter an einer gewünschten Stelle an einem Körper positioniert, wobei Positionsdaten der Marker durch IR-Kameras 2a erfasst werden. Das Planungssystem 1 ermittelt für eine vorgegebene durchtufiihrende Infusion unter Verwendung der vom Kemspinresonanzsystem 3 ermittelten Patientenparameter die geeigneten Kathetezparameter und Infusionsparameter, um die Infusion durchzuführen.

## Patentansprüche

1. Verfahren zur Planung einer Infusion in Lebergewebe, wobei
a) anatomische und/oder physiologische Patientendaten der Leber oder im Bereich der Leber individuell erfasst und
b) Patientenparameter aus den erfassten Patientendaten gewonnen werden und
c) die durchzuführende Infusion unter Verwendung der erfassten Patientendaten geplant wird,
**dadurch gekennzeichnet, dass**
d) berechnet wird wie sich eine verabreichte physiologisch wirksame Substanz im Lebergewebe verteilt und/oder physiologische Eigenschaften, insbesondere die Durchblutung, beeinflusst und
e) basierend auf dem Berechnungsergebnis die Verteilung eines anschließend zu verabreichenden therapeutischen Mittels berechnet wird und
f) basierend auf den Berechnungen in den Schritten d) und e) die Infusion des therapeutischen Mittels geplant wird.

2. Verfahren nach Anspruch 1, wobei die Patientendaten durch ein Kemspinresonanzverfahren (MRI), ein Computertomographieverfahren (CT), ein Röntgenverfahren, PET, SPECT oder ein Ultraschallverfahren erfasst werden.

3. Verfahren nach Anspruch 1 oder 2, wobei Informationen zum Blutdruck oder zur Blutdruckverteilung, zur Gewebestruktur, Gewebedichte, Durchblutung und/oder Stoffwechseleigenschaften des Gewebes als Patientenparameter verwendet werden.

4. Verfahren nach einem der vorhergehenden Ansprüche, wobei Substanz- oder Infusionsmittelparameter, welche chemische, biologische und/oder physikalische Eigenschaften des Infusionsmittels definieren, zur Planung der Infusion verwendet werden.

5. Verfahren nach einem der vorhergehenden Ansprüche, wobei Katheterparameter zur Planung der Infusion verwendet werden.

6. Verfahren nach einem der vorhergehenden Ansprüche, wobei eine Simulation der Infusionsmittelverteilung basierend auf den Patientenparametern, Katheterparametern und/oder Infusionsmittelparametern unter Verwendung eines mathematischen Modells durchgeführt wird.

7. Verfahren nach einem der vorhergehenden Ansprüche, wobei das mathematische Modell Eigenschaften oder Parameter der zu injizierenden Substanz, patientenbezogene Daten, Daten aus einem Behandlungsprotokoll und/oder Daten aus einer Datenbank verwendet, um die Infusionsmittelverteilung zu berechnen.

8. Verfahren nach einem der vorhergehenden Ansprüche, wobei das dynamische Verhalten des Körpers oder Gewebes, insbesondere bei stark durchbluteten Bereichen, bei der Planung oder Simulation berücksichtigt wird.

9. Verfahren nach einem der vorhergehenden Ansprüche, wobei ein Zielvolumen und/oder eine Infusionsmittelverteilung im Patienten vorgegeben wird und basierend darauf hierfür erforderliche Katheterparameter und Infusionsmittelparameter ermittelt werden.

10. Verfahren nach einem der vorhergehenden Ansprüche, wobei ein anatomisches oder patientenbezogenes Bild zur Darstellung der Substanzverteilung aus dem mathematischen Modell errechnet wird.

11. Verfahren nach einem der vorhergehenden Ansprüche, wobei ein Wirkungsbereich der mit den Planungsparametern infundierten Substanz simuliert wird.

12. Computerprogramm, welches in den Speicher eines Computers geladen werden kann und Softwarecodeabschnitte umfasst, mit welchen die Schritte gemäß einem der vorhergehenden Ansprüche ausgeführt werden, wenn das Programm auf einem Computer läuft.

13. Computerprogrammprodukt, das auf einem computergeeignetem Medium gespeichert ist und das Computerprogramm nach dem vorhergehenden Anspruch umfasst.

14. Vorrichtung zur Planung einer Infusion in Lebergewebe mit einem Patientendatenerfassungssystem (3) und einem ein Computerprogramm gemäß Anspruch 12 umfassenden Computersystem (1) zur Durchführung der Planung ei-ner Infusion von mindestens zwei verschiedenen nacheinander zu verabreichenden Substanzen basierend auf den erfassten Patientendaten gemäß Anspruch 1, welches mit dem Patientendatenerfassungssystem (3) verbunden ist.

15. Vorrichtung nach Anspruch 14 mit einem Navigationssystem (2) zum Positionieren mindestens jeweils eines Katheters für die Verabreichung eines physiologisch wirksamen Mittels und die Verabreichung eines therapeutischen Mittels basierend auf den Planungsdaten.

## Claims

1. A method for planning an infusion into hepatic tissue, wherein:
a) anatomical and/or physiological patient data of the liver or in the region of the liver are captured individually; and
b) patient parameters are obtained from the captured patient data; and
c) the infusion to be performed is planned using the captured patient data,
**characterised in that**
d) the method calculates how an administered, physiologically active substance is distributed in the hepatic tissue and/or how it influences physiological properties, in particular blood flow, and
e) calculates the distribution of a therapeutic agent to be administered subsequently, based on the calculation result, and
f) plans the infusion of the therapeutic agent, based on the calculations in Steps d) and e).

2. The method according to claim 1, wherein the patient data are captured using a nuclear spin resonance (MRI) method, a computer tomography (CT) method, an x-ray method, PET, SPECT, or an ultrasound method.

3. The method according to claim 1 or 2, wherein information relating to the blood pressure or blood pressure distribution, tissue structure, tissue density, blood flow and/or metabolic properties of the tissue is used as patient parameters.

4. The method according to any one of the preceding claims, wherein substance or infusion agent parameters which define chemical, biological and/or physical properties of the infusion agent are used for planning the infusion.

5. The method according to any one of the preceding claims, wherein catheter parameters are used for planning the infusion.

6. The method according to any one of the preceding claims, wherein the distribution of the infusion agent is simulated based on the patient parameters, catheter parameters and/or infusion agent parameters, using a mathematical model.

7. The method according to any one of the preceding claims, wherein the mathematical model uses properties or parameters of the substance to be injected, patient-related data, data from a treatment report and/or data from a database, in order to calculate the distribution of the infusion agent.

8. The method according to any one of the preceding claims, wherein the dynamic behaviour of the body or tissue, in particular in regions with high blood flow, is taken into account when planning or simulating.

9. The method according to any one of the preceding claims, wherein a target volume and/or a distribution of the infusion agent in the patient are pre-set, and based on this, catheter parameters and infusion agent parameters necessary for this are ascertained.

10. The method according to any one of the preceding claims, wherein an anatomical or patient-related image for displaying the distribution of the substance is calculated from the mathematical model.

11. The method according to any one of the preceding claims, wherein a region of effectiveness of the substance infused using the planning parameters is simulated.

12. A computer program which can be loaded into the memory of a computer and includes software code sections using which the steps in accordance with any one of the preceding claims are performed, when the program is running on a computer.

13. A computer program product which is stored on a computer-compatible medium and includes the computer program according to the preceding claim.

14. A device for planning an infusion into hepatic tissue, comprising a patient data capture system (3) and a computer system (1) which includes a computer program in accordance with claim 12, for planning an infusion of at least two different substances to be administered successively, based on the captured patient data in accordance with claim 1, and which is connected to the patient data capture system (3).

15. The device according to claim 14, comprising a navigation system (2) for positioning at least one respective catheter for administering a physiologically active agent and for administering a therapeutic agent, based on the planning data.

## Revendications

1. Procédé pour programmer une perfusion dans un tissu hépatique, dans lequel
a) les données anatomiques et/ou physiologiques du patient sont relevées individuellement dans le foie ou dans la région du foie et
b) des paramètres du patient sont obtenus à partir des données relevées du patient et
c) la perfusion à exécuter est programmée par utilisation des données relevées du patient,
**caractérisé en ce que**
d) on calcule comment une substance active physiologiquement administrée se répartit dans le tissu hépatique et/ou des propriétés physiologiques, en particulier la circulation sanguine, sont influencées et
e) sur la base du résultat du calcul, on calcule la distribution d'une substance thérapeutique à administrer ensuite, et
f) sur la base des calculs des états d) et e), on programme la perfusion de la substance thérapeutique.

2. Procédé selon la revendication 1, dans lequel les données du patient sont relevées par un procédé de résonance magnétique (MRI), un procédé de tomographie par ordinateur (CT), un procédé radiographique, PET, SPECT ou un procédé aux ultrasons.

3. Procédé selon la revendication 1 ou 2, dans lequel on utilise des informations relatives à la pression sanguine ou à la distribution de la pression sanguine, à la structure du tissu, à la densité du tissu, à la circulation sanguine et/ou des propriétés du métabolisme du tissu en tant que paramètres du patient.

4. Procédé selon l'une des revendications précédentes, dans lequel on utilise des paramètres de la substance ou du produit de perfusion, qui définissent des propriétés chimiques, biologiques et/ou physiques du produit de perfusion, pour programmer la perfusion.

5. Procédé selon l'une des revendications précédentes, dans lequel on utilise des paramètres de cathéter pour programmer la perfusion.

6. Procédé selon l'une des revendications précédentes, dans lequel on procède à une simulation de la distribution d'un produit de perfusion sur la base des paramètres du patient, des paramètres de cathéter et/ou des paramètres de produit de perfusion en utilisant un modèle mathématique.

7. Procédé selon l'une des revendications précédentes, dans lequel on utilise le modèle mathématique, des propriétés ou paramètres de la substance à injecter, des données relatives au patient, des données provenant d'un protocole de traitement et/ou des données provenant d'une banque de données, pour calculer la distribution du produit de perfusion.

8. Procédé selon l'une des revendications précédentes, dans lequel on prend en compte le comportement dynamique du corps ou du tissu, en particulier dans le cas de régions fortement irriguées en sang, pour la programmation ou la simulation.

9. Procédé selon l'une des revendications précédentes, dans lequel on prédéfinit un volume cible et/ou une distribution du produit de perfusion dans le patient, et sur la base de ceci on détermine les paramètres de cathéter et les paramètres de produit de perfusion nécessaires pour cela.

10. Procédé selon l'une des revendications précédentes, dans lequel on calcule, à partir du modèle mathématique, une image anatomique ou relative au patient pour représenter la distribution de la substance.

11. Procédé selon l'une des revendications précédentes, dans lequel on simule une zone d'action de la substance infusée, avec les paramètres de programmation.

12. Programme d'ordinateur qui peut être chargé dans la mémoire d'un ordinateur et comprend des segments de code de logiciel avec lesquels peuvent être exécutées les étapes selon l'une des revendications précédentes, lorsque le programme tourne sur un ordinateur.

13. Produit de programme d'ordinateur qui est mémorisé sur un support compatible avec un ordinateur et comprend le programme d'ordinateur selon la revendication précédente.

14. Dispositif pour programmer une perfusion dans le tissu hépatique comportant un système de relevé (3) de données d'un patient ainsi qu'un système d'ordinateur (1) comprenant un programme d'ordinateur selon la revendication 12, pour mettre en oeuvre la programmation d'une perfusion d'au moins deux substances différentes à administrer l'une après l'autre, sur la base des données relevées du patient selon la revendication 1, lequel système d'ordinateur est relié au système de relevé (3) des données du patient.

15. Dispositif selon la revendication 14 comportant un système de navigation (2) pour positionner au moins chaque fois un cathéter pour administrer une substance active physiologiquement et administrer une substance thérapeutique sur la base des données de la programmation.
